# EUROPEAN PATENT SPECIFICATION

(11) **EP 0 355 035 B1**
(45) Date of publication and mention of the grant of the patent: **18.05.1994**
(21) Application number: 89117774.3
(22) Date of filing: 03.11.1987
(51) Int. Cl.: A61B 17/58

(54) **Bone plate with conical holes**
Knochenplatte mit konischen Löchern
Plaque d'ostéosynthèse à trous coniques

(43) Date of publication of application: 21.02.1990
(62) Divisional of application: 88900013.9
(73) Proprietor: SYNTHES AG, Chur, 7002 Chur (CH)
(72) Inventor: Tepic, Slobodan, CH-7270 Davos (CH); Perren, Stephen M., CH-7260 Davos-Dorf (CH); Sutter, Franz, CH-4435 Niederdorf (CH); Straumann, Fritz, CH-4437 Waldenburg (CH)
(74) Representative: Lusuardi, Werther Giovanni, Dr.

(56) References cited:
- EP-A- 0 048 038
- WO-A-87/00419
- DE-A- 2 013 968
- DE-A- 2 438 669
- DE-A- 2 548 077
- DE-A- 3 027 138
- DE-C- 3 442 004
- FR-A- 2 501 033
- US-A- 3 779 240
- Taschenbuch für den Maschinenbau, page 405, Springer Verlag 1986
- Metall-Bearbeitung, Verlag Otto Walter, page 392

## Description

### Background of the Invention

This invention relates to an implant for osteosynthesis, comprising a device connectable with a bone with at least two clearance holes and screws with a head for fastening said device rigidly to said bone.

In conventional operative treatment of bone fractures a plate is applied to fractured bone bridging the fracture. The plate is fixed to the bone by means of a number of screws which are tightened in the bone producing compressive stresses between the plate and the bone. Transmission of functional load from the bone to the plate and back to the bone is achieved mostly by means of friction corresponding to the compressive stresses. Both the near and the far wall of the bone cortex are usually engaged by the screws. Bone cortex is supplied by blood from periosteal, or outer side, and from endosteal, or inner side. Compression between the plate and bone impedes the blood perfusion of the cortex region under the plate. This is believed to increase the chances of infection - a major complication of operative fracture treatment. Dead bone under the plate is in due course remodelled and revascularized. Porosity within the remodelling bone persists for a long time and reduces bone strength, particularly in fatigue. This requires keeping the plate on bone longer than needed for the fracture to heal.

The use of long screws running first through the near cortex, then through the medullary canal, and through the far cortex may further compromise blood supply of the fractured bone by cutting through larger blood vessels.

### Summary of the Invention

The invention as claimed is intended to remedy these drawbacks. It solves the problem of how to design an implant for osteosynthesis that allows operative treatment of bone fractures with minimal vascular damage to the treated bone.

Further advantages offered by the invention are - besides the reduced damage to bone and in particular to the intramedullary vascular system - the possibility to use short screws which can be made self-tapping without compromises necessary with long self-tapping screws. Furthermore the plate design according to the invention allows for doubling the strength of the bone plate due to the reduced screw hole size. Variation of strength along the bone plate according to the invention is much smaller than in conventional plates. The same is true for stiffness. This facilitates plate adaptation to curved bone surfaces.

The various features of novelty which characterize the invention are pointed out with particularity in the claims annexed to and forming part of this disclosure. For the better understanding of the invention, its operating advantages and specific objects attained by its use, reference should be had to the accompanying drawings and descriptive matter in which are illustrated and described preferred embodiments of the invention.

### Brief Description of the Drawings

In the drawings:
Fig. 1 is a schematic view of a fractured bone with a conventional bone plate.
Fig. 2 is a cross section of the plate according to Figure 1.
Fig. 3 is a transverse cross section of a bone plate according to the invention with short screws.
Fig. 4 is a longitudinal cross section of a bone plate according to Figure 3.
Fig. 5 is a cross section of a bone plate according to the invention with resorbable buds at the plate undersurface.
Fig. 6 is a cross section of a bone plate according to the invention with an expandable spherical insert.
Fig. 7 is a cross section of a bone plate with an expandable conical insert.
Fig. 8 is a cross section of a bone plate according to the invention with removable clip-on springs.

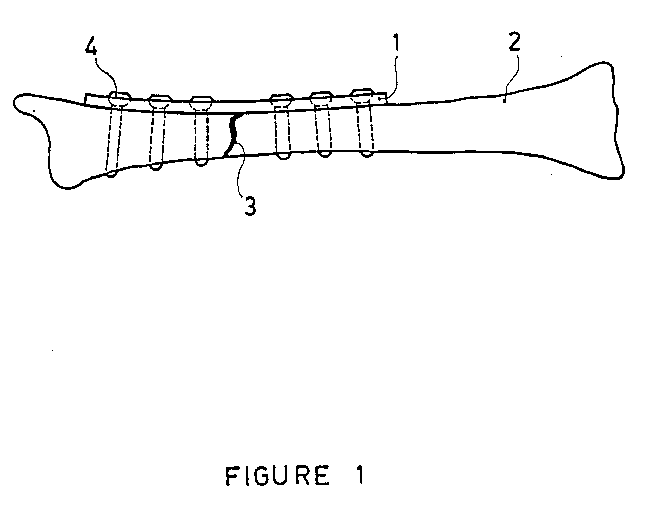

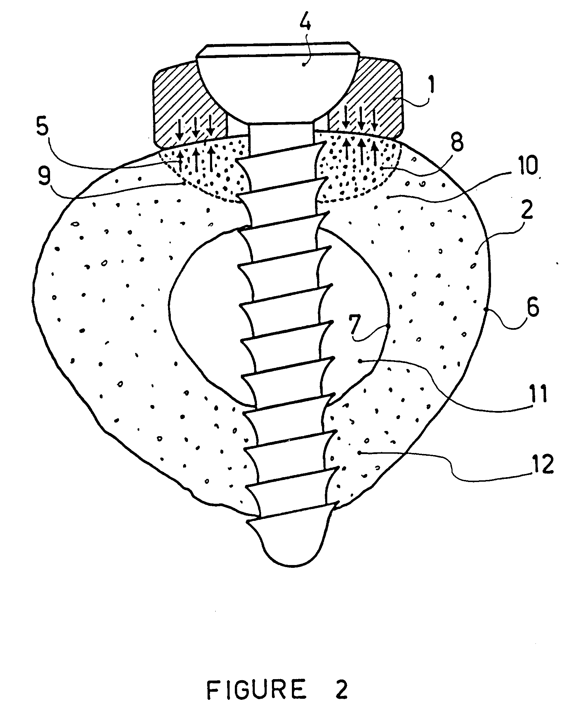

Fig. 1 represents a conventional bone plate 1 for the treatment of bone fractures fixed to the bone 2 by means of a number of screws 4. Screws 4 are tightened in the bone 2 producing compressive stresses 5 between the plate 1 and the bone 2. As shown in Fig. 2 transmission of functional load from the bone 2 to the conventional plate 1 and back to the bone 2 is achieved mostly by means of friction corresponding to the compressive stresses 5. Both the near and the far wall of the bone cortex 2 are usually engaged by the screws 4. Bone cortex 2 is supplied by blood from periosteal, or outer side 6, and from endosteal, or inner side 7. Compression 5 between the conventional plate 1 and bone 2 impedes the blood perfusion of the cortex region 8 under the conventional plate 1. This is believed to increase the chances of infection - a major complication of operative fracture treatment. Dead bone within region 8 is in due course remodelled and revascularized, whereby remodelling activity starts at periphery 9 of unperfused bone and proceeds towards the plate 1. Porosity within the remodelling bone persists for a long time and reduces bone strength, particularly in fatigue. This requires keeping the conventional plate 1 on bone longer than needed for the fracture to heal. Long screws 4 in running first through the near cortex 10, then through the medullary canal 11, and through the far cortex 12 may further compromise blood supply of the fractured bone by cutting through larger blood vessels.

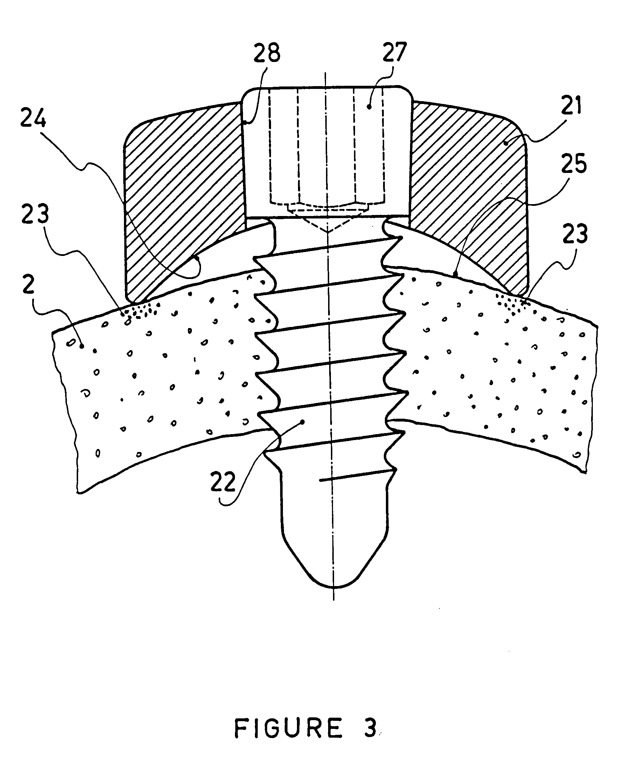

In Figure 3 the plate 21 according to the invention is applied to the bone 2 by means of short screws 22. Using short screws 22 in place of conventional long ones is made possible by rigidly locking the screw 22 in the plate 21. To this purpose the screws 22 are provided with a conical head 27 which upon insertion locks safely in the conical hole 28 of the plate 21. The angle of the conus on the screw head 27 is smaller than the resulting friction angle, preferably in the range corresponding to a cone taper of 1:5 to 1:20. The locking between plate 21 and screws 22 prevents tilting of the screws 22 within the cortex 2. Loads between the bone 2 and the plate 21 are transferred directly through the screw 22, which now act as pegs (under shear) rather than anchors (under tension). In addition to reducing damage to bone 2 and in particular to the intramedullary vascular system, short screws 22 can be made self-tapping without compromises necessary with long self-tapping screws.

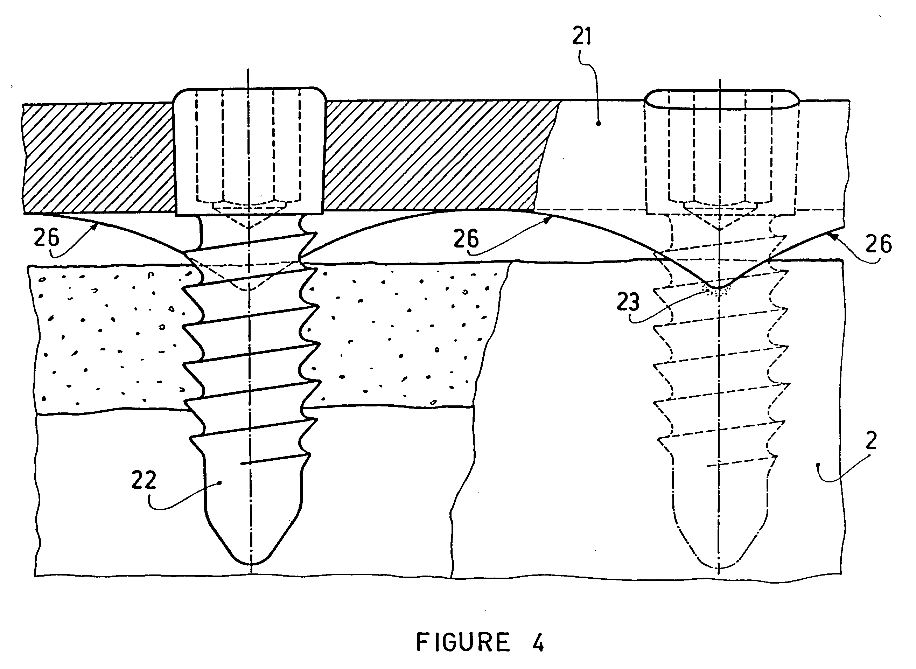

In the preferred embodiment according to Fig. 3 the plate undersurface is shaped so as to permit only point contact to the bone at very small areas 23. This may be achieved by arching the underside of the plate 24 at a curvature larger than that of the bone outer contour 25. In the longitudinal direction (Figure 4), the plate underside is also shaped with multiple arches 26 spanning the distance between the screws 22. This leaves the possibility of contact only at small areas 23.

The described undercutting of the plate 21 with arches 24 in transverse and arches 26 in longitudinal direction may be used to achieve other advantages in addition to reducing the plate-bone contact to point-like areas 23. Conventional plate strength is lowest at the screw hole sections and so is the plate stiffness. Overall plate strength is limited by the weakest section. Keeping the outer dimensions similar to the normal plate, the plate design according to the invention allows for doubling the strength. This is mostly due to the reduced screw hole size. Variation of strength along the plate is much smaller than in conventional plates. The same is true for stiffness. This facilitates plate adaptation to curved bone surfaces.

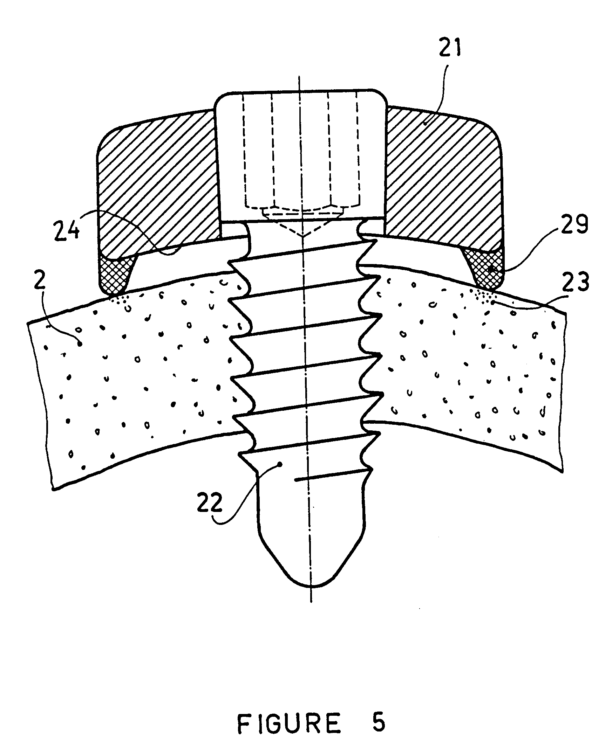

Figure 5 shows another way to reduce the contact between the plate 21 and the bone 2 to point- like areas 23. Small buds 29 are added to the plate undersurface. These may be made from a material which is resorbable, or even dissoluble in body (e.g. polysaccharides), since they need to support the plate 21 only during screw insertion and locking into the plate 21.

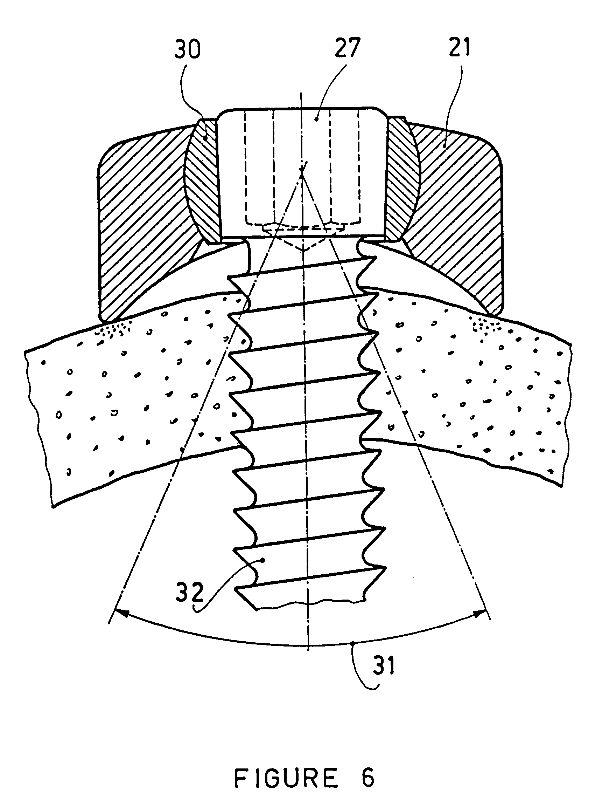

Figure 6 shows a plate 21 according to the invention combined with a long screw 32 which may be locked into the plate 21 via the spherical insert 30 with substantial freedom of angular placement 31. The conical head 27 of the screw 32 expands the slotted sphere 30, locking itself and the sphere 30 in the plate 21. Taper on the conus is again smaller than friction angle, preferably between 1:5 and 1:20.

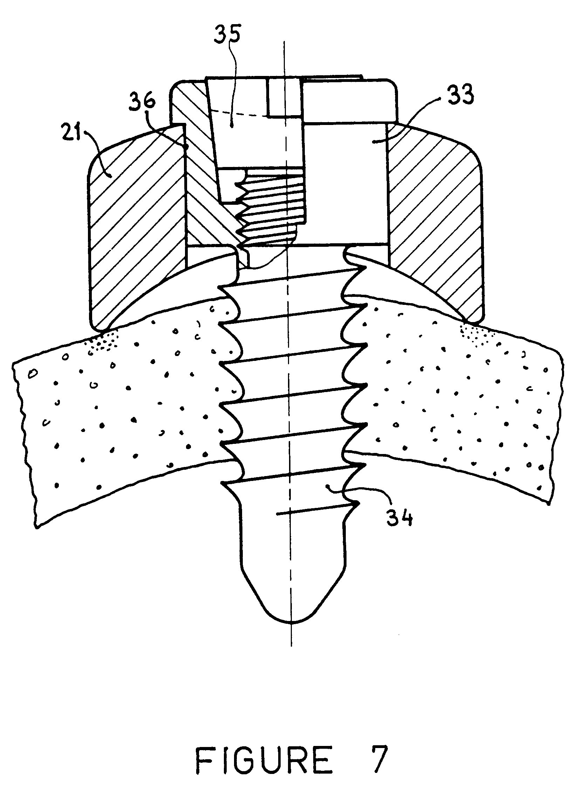

Figure 7 shows another implant where the locking of the screw 34 in the plate 21 is achieved by the conical insert 35 which expands the slotted screw head 33 thereby forming a rigid connection at the cylindrical interface 36.

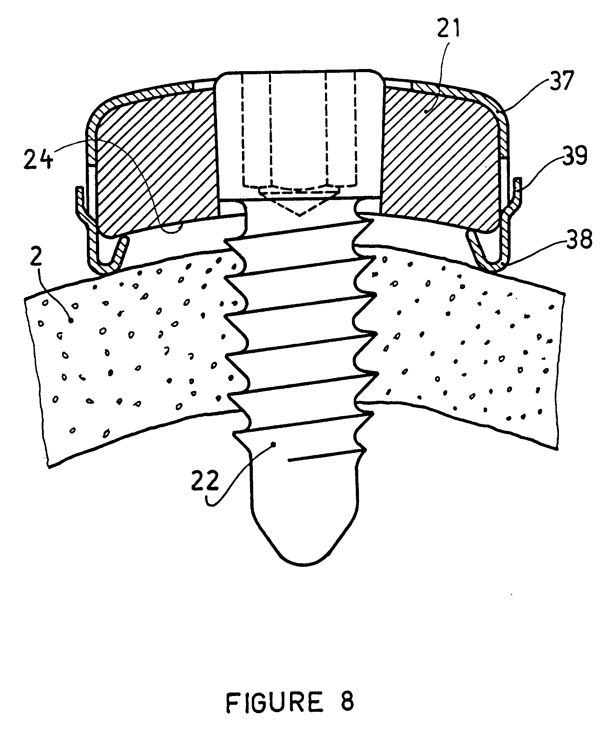

Figure 8 shows a further embodiment of the invention where the minimal plate contact to the bone 2 is achieved through removable clip-on springs 37 providing bud-like extensions 38 on the undersurface of plate 21. Springs 37 may be removed following insertion and locking of screw 22 in the plate 21. Hooks 39 facilitate removal following screw tightening.

## Claims

1. An implant for osteosynthesis, comprising a device (21) connectable with a bone (2) with at least two clearance holes (28) and screws (22) with a head (27) for fastening said device (21) rigidly to said bone (2), characterized in that said clearance holes (28) have the shape of a cone for receiving a correspondingly shaped screw head (27), whereby said cone has a cone angle smaller than the resulting friction angle, preferably in the range corresponding to a cone taper of 1:5 to 1:20.

2. Implant according to claim 1, characterized in that an intermediate body (30) is provided fitting in said clearance hole (28) which is expandable by the conical screw head (27) having a cone angle being smaller than the resulting friction angle, preferably in the range corresponding to a cone taper of 1:5 to 1:20.

3. Implant according to claim 1 or 2, characterized in that said screws (22) have a reduced length for engagement only with one cortex of said bone (2).

4. Implant according to one of the claims 1 to 3, characterized in that said screws (22) are self-tapping.

5. Implant according to one of the claims 1 to 4, characterized in that the contact surface (23) of said device (21) to said bone (2) is reduced to the level necessary only for initially locking said screws (22) to said device (21) at the time of insertion.

6. Implant according to one of the claims 1 to 5, characterized in that the contact surface of the underside (24) of said device (21) to said bone (2) is reduced to point-like areas (23).

7. Implant according to one of claims 1 to 6, characterized in that the contact surface of the underside (24) of said device (21) to said bone (2) is less than 5%, preferably less than 2% of the full undersurface area (24) of said device (21).

8. Implant according to one of the claims 1 to 7, characterized in that the contact surface of the underside (24) of said device (21) to said bone (2) consists of contact buds (29) made of a material which is resorbable or dissoluble in body fluids, preferably in the form of resorbable or dissoluble buds (29) applied on the normal unstructured bone contact surface of said device (21).

9. Implant according to one of the claims 1 to 8, characterized in that additional clip-on springs (37) are provided for mounting on said device (21) which form bud-like extensions (38) on the undersurface (24) of said device (21).

10. Implant according to one of the claims 1 to 9, characterized in that said device (21) is of plate-like form.

11. Implant according to one of the claims 1 to 10, characterized in that said screw heads (27) are expandable by means of a body (35) insertable into said head (27).

12. Implant according to claim 1 or 11, characterized in that at least one of said clearance holes (28) is designed as a self compressing hole.

13. Implant according to one of the claims 1 to 12, characterized in that only one of said clearance holes (28) is designed as self compressing hole.

14. Implant according to one of the claims 1 to 13, characterized in that the contact surface (23) of said device (21) to said bone (2) is reduced to be less than the full undersurface area of said device (21).

## Patentansprüche

1. Implantat für die Osteosynthese mit einer mit einem Knochen (2) verbindbaren Vorrichtung (21) mit mindestens zwei Durchgangslöchern (28) und Schrauben (22) mit einem Kopf (27) zur rigiden Befestigung der Vorrichtung (21) an den Knochen (2), dadurch gekennzeichnet, dass die Durchgangslöcher (28) die Form eines Konus besitzen um einen entsprechend geformten Schraubenkopf (27) aufzunehmen, wobei der Konus einen Konuswinkel besitzt, welcher kleiner als der resultierende Reibungswinkel ist, vorzugsweise in einem Bereich, der einer Konizität von 1:5 bis 1:20 entspricht.

2. Implantat nach Anspruch 1, dadurch gekennzeichnet, dass ein Zwischenelement (30) vorgesehen ist, welches in das Durchgangsloch (28) passt und das mittels des konischen Schraubenkopfes (27) aufweitbar ist, welcher einen Konuswinkel besitzt, der kleiner als der resultierende Reibungswinkel ist, vorzugsweise in einem Bereich, der einer Konizität von 1:5 bis 1:20 entspricht.

3. Implantat nach Anspruch 1 oder 2, dadurch gekennzeichnet, dass die Schrauben (22) eine reduzierte Länge aufweisen, so dass sie nur in eine Kortex des Knochens (2) eingreifen.

4. Implantat nach einem der Ansprüche 1 - 3, dadurch gekennzeichnet, dass die Schrauben (22) selbstschneidend sind.

5. Implantat nach einem der Ansprüche 1 - 4, dadurch gekennzeichnet, dass die Kontaktfläche (23) der Vorrichtung (21) mit dem Knochen (2) auf dasjenige Ausmass reduziert ist, welches die anfängliche Fixierung der Schrauben (22) an die Vorrichtung (21) zur Zeit der Implantation nötig macht.

6. Implantat nach einem der Ansprüche 1 - 5, dadurch gekennzeichnet, dass die Kontaktfläche der Unterseite (24) der Vorrichtung (21) mit dem Knochen (2) zu punktähnlichen Bereichen (23) reduziert ist.

7. Implantat nach einem der Ansprüche 1 - 6, dadurch gekennzeichnet, dass die Kontaktfläche der Unterseite (24) der Vorrichtung (21) mit dem Knochen (2) kleiner als 5 %, vorzugsweise kleiner als 2 % der vollen Fläche der Unterseite (24) der Vorrichtung (21) ist.

8. Implantat nach einem der Ansprüche 1 - 7, dadurch gekennzeichnet, dass die Kontaktfläche der Unterseite (24) der Vorrichtung (21) mit dem Knochen (2) aus Kontaktknospen (29) besteht, welche aus einem resorbierbaren oder in Körperflüssigkeiten löslichen Material besteht, vorzugsweise in Form von resorbierbaren oder löslichen Kontaktknospen (29), welche auf der normalen, unstrukturierten Knochenkontaktfläche der Vorrichtung (21) angebracht sind.

9. Implantat nach einem der Ansprüche 1 - 8, dadurch gekennzeichnet, dass zusätzliche, einrastbare Federn (37) zur Montage auf der Vorrichtung (21) vorgesehen sind, welche knospenähnliche Fortsätze (38) auf der Unterseite (24) der Vorrichtung (21) bilden.

10. Implantat nach einem der Ansprüche 1 - 9, dadurch gekennzeichnet, dass die Vorrichtung (21) plattenförmig ist.

11. Implantat nach einem der Ansprüche 1 - 10, dadurch gekennzeichnet, dass die Schraubenköpfe (27) mittels eines in die Schraubenköpfe (27) einführbaren Körpers (35) aufweitbar sind.

12. Implantat nach einem der Ansprüche 1 - 11, dadurch gekennzeichnet, dass mindestens eines der Durchgangslöcher (28) als selbstkomprimierendes Loch ausgebildet ist.

13. Implantat nach einem der Ansprüche 1 - 12, dadurch gekennzeichnet, dass nur eines der Durchgangslöcher (28) als selbstkomprimierendes Loch ausgebildet ist.

14. Implantat nach einem der Ansprüche 1 - 13, dadurch gekennzeichnet, dass die Kontaktfläche (23) der Vorrichtung (21) mit dem Knochen (2) soweit reduziert ist, dass sie kleiner als die volle Fläche der Unterseite (24) der Vorrichtung (21) ist.

## Revendications

1. Implant d'ostéosynthèse, comportant un dispositif (21) pouvant être relié à un os (2), avec au moins deux trous de passage (28) et des vis (22) à tête (27) en vue de solidariser ledit dispositif (21) avec ledit os (2), caractérisé en ce que lesdits trous de passage (28) possèdent la forme d'un cône de réception d'une tête de vis (27) de forme correspondante, tandis que ledit cône possède un angle de cône inférieur à l'angle de friction résultant, et est de préférence situé dans la plage correspondant à un rétrécissement de cône de 1:5 à 1:20.

2. Implant selon la revendication 1, caractérisé en ce qu'un corps intermédiaire (30) est prévu pour s'ajuster dans ledit trou de passage (28), ce corps intermédiaire (30) étant expansible par la tête conique (27) de la vis qui possède un angle de cône plus petit que l'angle de friction résultant, et est de préférence situé dans la plage correspondant à un rétrécissement de cône de 1:5 à 1:20.

3. Implant selon la revendication 1 ou 2, caractérisé en ce que lesdites vis (22) ont une longueur réduite pour ne s'accrocher qu'à un cortex dudit os (2).

4. Implant selon une des revendications 1 à 3, caractérisé en ce que lesdites vis (22) sont autotaraudeuses.

5. Implant selon une des revendications 1 à 4, caractérisé en ce que la surface de contact (23) dudit dispositif (21) avec ledit os (2) est réduite au niveau nécessaire pour bloquer initialement lesdites vis (22) audit dispositif (21) au moment de l'insertion.

6. Implant selon une des revendications 1 à 5, caractérisé en ce que la surface de contact de la face inférieure (24) dudit dispositif (21) avec ledit os (2) est réduite à des zones (23) quasi ponctuelles.

7. Implant selon une des revendications 1 à 6, caractérisé en ce que la surface de contact de la face inférieure (24) dudit dispositif (21) avec ledit os (2) est de moins de 5%, de préférence de moins de 2% de la totalité de la superficie de la surface inférieure (24) dudit dispositif (21).

8. Implant selon une des revendications 1 à 7, caractérisé en ce que la surface de contact de la face inférieure (24) dudit dispositif (21) avec ledit os (2) est constituée de pastilles de contact (29) réalisées en un matériau qui peut être résorbé ou se dissout dans les fluides corporels, présentant de préférence la forme de pastilles (29) susceptibles de résorption ou solubles disposées sur la surface de contact avec l'os, normale et non structurée, dudit dispositif (21).

9. Implant selon une des revendications 1 à 8, caractérisé en ce que des ressorts (37) de serrage supplémentaires sont prévus pour être montés sur ledit dispositif (21) et forment des saillies (38) en forme de pastilles sur la surface inférieure (24) dudit dispositif (21).

10. Implant selon une des revendications 1 à 9, caractérisé en ce que ledit dispositif (21) est en forme de plaque.

11. Implant selon une des revendications 1 à 10, caractérisé en ce que lesdites têtes (27) de vis peuvent être dilatées au moyen d'un corps (35) pouvant être inséré dans ladite tête (27).

12. Implant selon la revendication 1 ou 11, caractérisé en ce qu'au moins un desdits trous de passage (28) est conçu comme trou à autocompression.

13. Implant selon une des revendications 1 à 12, caractérisé en ce que seul un desdits trous de passage (28) est conçu comme trou à autocompression.

14. Implant selon une des revendications 1 à 13, caractérisé en ce que la surface de contact (23) dudit dispositif (21) avec ledit os (2) est réduite de manière à être inférieure à la superficie totale de la surface inférieure dudit dispositif (21).
